# EUROPEAN PATENT SPECIFICATION

(11) **EP 3 398 546 B1**
(45) Date of publication and mention of the grant of the patent: **19.10.2022**
(21) Application number: 16880762.6
(22) Date of filing: 31.10.2016
(51) Int. Cl.: A61B 18/02, A61M 25/10

(54) **TUMOR CRYOABLATION CATHETER**
TUMORKRYOABLATIONSKATHETER
CATHÉTER DE CRYOABLATION TUMORALE

(30) Priority: 31.12.2015 CN 201511008633
(43) Date of publication of application: 07.11.2018
(73) Proprietor: Ningbo Senscure Biotechnology Co., Ltd., Ningbo, Zhejiang 315336 (CN)
(72) Inventor: LV, Shiwen, Ningbo Zhejiang 315336 (CN); JIANG, Haowen, Ningbo Zhejiang 315336 (CN); XING, Zongjiang, Ningbo Zhejiang 315336 (CN); QI, Dibo, Ningbo Zhejiang 315336 (CN); DIAO, Yuepeng, Ningbo Zhejiang 315336 (CN)
(74) Representative: Noble, Nicholas
(86) International application number: PCT/CN2016/103992
(87) International publication number: WO 2017/113971

(56) References cited:
- EP-A2- 2 387 964
- WO-A1-2011/082278
- WO-A1-2012/058153
- CN-A- 104 905 873
- CN-A- 105 581 838
- CN-U- 202 637 100
- CN-U- 204 797 985
- CN-U- 204 797 986
- US-A1- 2004 243 117
- US-A1- 2008 033 477
- US-A1- 2009 299 355
- US-A1- 2014 012 244
- US-A1- 2014 012 244
- US-A1- 2015 342 660
- US-B2- 8 579 890

## Description

### RELATED APPLICATIONS

The present invention application claims the benefit of priority of Chinese Patent Application No. 201511008633.8, filed on December 31, 2015 and entitled "New Tumor Cryoablation Catheter".

### TECHNICAL FIELD

The present invention belongs to the field of medical instruments, and more particularly, to a new tumor cryoablation catheter according to appended claim 1.

### BACKGROUND

According to a report from the World Health Organization, in 2008, there were approximately 12.7 million new cancer patients worldwide, and 7.6 million patients died of cancer, especially in developing countries, the number of new cancer patients accounts for 56%. According to "2012 China Cancer Registration Annual Report" issued by the National Cancer Registry of China, there were about 3.5 million new cancer patients each year in China, and about 2.5 million people died of cancer. It is worrying that the cancer incidence and the cancer morbidity will continue to climb in China in the next 10 years. It is speculated that by 2020, the global cancer incidence will increase by 50%, that is, 15 million cancer patients will be newly increased each year. What's more, the death toll of cancer is also rising rapidly around the world, and the number is likely to increase to 13.2 million in 2030.

Only about 20% of tumor patients can receive surgical resection, therefore minimally invasive treatment techniques such as cryoablation, radiofrequency ablation, microwave ablation, laser thermotherapy and so on, are becoming the frontier of contemporary clinical medicine, and cryoablation is one representative technology among them.

Cryoablation treatment involves the application of the complex cooling systems to properly freeze the target biological tissues to be treated under very low temperatures. In many of these systems, a cryoprobe or a cryocatheter with particular shape and size is provided and configured to contact part of the selected tissues, without affecting adjacent healthy tissues or organs. Extreme freezing is produced by some kinds of refrigerants which are introduced through the distal end of the cryoprobe. The distal end of the cryoprobe must be in direct thermal contact with the target biological tissues to be treated. Cryotherapy is a treatment method whose main principle is to destroy or remove the living tissues under control through freezing local tissues, or it is referred to as cryosurgery. The tissues are rapidly frozen, and the temperature drops below 0°C, then the tissue fluid inside and outside the cells forms ice crystals, and the structures of cells are destroyed. Following cell dehydration, lipoprotein of the membrane system degenerates, and ischemic infarct occurs in the tissues, then the tissues lack nutrient and eventually become necrotic. In the process of rewarming, the destroyed proteins of the tissues have new antigenic properties that stimulate the body's immune system and produce an autoimmune response. Therefore, when cryotherapy is applied to treat local primary malignant tumors, the growth of distant metastatic tumors may be inhibited. The refrigerant may be spread on the site to be treated during treatment, or the freezing portion of the metal (or the balloon) contacts with local tissues or penetrates into local tissues.

The currently available cryoablation systems are mainly divided into two major categories: one is the cold knife in the liquid nitrogen system, which is based on the principle that substance absorbs a large amount of latent heat of vaporization when changing from liquid to gas, to make the tissues cool down quickly. Liquid nitrogen has a desired low temperature of approximately 200°C below zero, and when the liquid nitrogen is introduced to the cryoprobe and is in thermal contact with the surrounding warm biological tissues, the temperature of the liquid nitrogen increases above its boiling temperature (196°C below zero), and the liquid nitrogen rapidly absorbs heat from the distal end of the cryoprobe. The other one is the cryoprobe in the gas throttling system, which takes advantages of the temperature variation of the high pressure gas when it undergoes adiabatic throttle through small holes, so as to realize the cooling and warming treatment processes of the cryoablation, and the representative product is Argon-Helium knife.

Cryotherapy has fewer wounds and good curative effects, and the effective rate of cryotherapy is over 90%. Cryotherapy has become an indispensable therapy for a variety of cancers. Cryoablation can quickly reduce tumor burden, reduce suffering of patients, improve life quality of patients, and take an important role irreplaceable by radiotherapy and chemotherapy.

The cryoablation balloon catheter can make the damages to the diseased tissues more uniform and more thorough, thereby improving the long-term success rate and reducing recurrence. Compared with the radiofrequency ablation, the cryoablation can ensure the structural integrity of a cell, and protect the fiber connective tissue around the cells from being damaged, and ensure the surface of the cells at the site of cryoablation relatively glossy and smooth.

At present, one category of the cryoablation products for treating tumors are provided with metal probes. The metal probe is made of hard materials and its cryoablation range is rather small, which greatly reduces the application range of such products. The other category of cryoablation products are cryoablation balloon catheters. The cryoablation balloon catheter takes advantages of the characteristics of the balloon, and the effective freezing area is the side wall of the balloon; the balloon is made of plastic material. In operation, the balloon is filled and inevitably becomes deformed, and the wall of the balloon becomes a curved surface, therefore, when the curved surface contacts with the tissue wall, they are in point contact but not in surface contact, which has great influences on the cryoablation, and reduces the effective range of freezing.

The U.S. Patent Application Publication US20140058369A1 describes a Cryo-Ablation Refrigerant Distribution Balloon Catheter. This patent application provides a new structure for refrigerant entering the balloon in a variety of manners. According to this structure, the distal end of each balloon of the balloon catheter cannot be fixed, therefore the support at the distal end of the balloon, which is important, is not provided, and the balloon lacks strong rigidity. When the structure is inserted into the operation channel or some narrow lumens, or encounters resistance during going ahead, the balloon is easy to be deformed and folded, thus increasing the resistance to the inserting of the structure. Even more, the friction may cause the balloon and the catheter to break and be damaged, which increases the difficulty of operation and the risks in operation. At the same time, in operation, when the balloon presses against the wall of the tissues of the human body, the lack of support at the distal end of the balloon will cause the internal gas intake structure of the balloon to deviate from the center of the balloon, and make the distribution of the freezing temperature uneven, thus affecting the freezing effects.

The U.S. Patent Application Publication US20120016355A1 describes a cryoablation balloon catheter and a related method. A wire guiding channel is provided inside the balloon of the catheter and acts as a support for the balloon delivery, which solves the delivery problem of the balloon catheter to some extent. However, the design still has some defects. The design allows the center of the balloon at the distal end of the cryoablation catheter to be occupied by the wire guiding channel. When the refrigerant enters the balloon, the refrigerant inclines towards one side of the balloon, as a result, the freezing is uneven, and the freezing effect is affected. Moreover, the refrigerant directly enters the balloon, having greater impacts on the balloon, and so the safety requirement for the balloon is higher.

US2009/299355 is directed to a method of ablating body tissue includes (a) locating an inflatable balloon portion of a cryotherapy balloon catheter at a treatment site internal to a patient's body, and inflating the inflatable balloon portion; (b) employing electrodes that are disposed on an expandable surface of the inflatable balloon portion to electrically characterize body tissue at the treatment site; (c) ablating the body tissue by supplying a cryotherapy agent to the inflatable balloon portion to cool the body tissue to a therapeutic temperature; (d) employing the electrodes to determine whether the ablating caused desired electrical changes in the body tissue; and (e) repeating (c) and (d) when it is determined that the ablating did not cause the desired electrical changes.

US2014/012244 is directed to methods and apparatus for the treatment of a body cavity or lumen are described where a heated fluid and/or gas may be introduced through a catheter and into treatment area within the body contained between one or more inflatable/expandable members. The catheter may also have optional pressure and temperature sensing elements which may allow for control of the pressure and temperature within the treatment zone and also prevent the pressure from exceeding a pressure of the inflatable/expandable members to thereby contain the treatment area between these inflatable/expandable members. Optionally, a chilled, room temperature, or warmed fluid such as water may then be used to rapidly terminate the treatment session.

US2008/033477 is directed to a reinforced angioplasty balloon having an external elastic sheath. The elastic sheath maintains, along its length, a substantially uniform circular cross-section during balloon inflation and forces a deflated balloon into a compacted, substantially circular, wingless profile.

WO2012/058153 is directed to neuromodulation cryotherapeutic devices and associated systems and methods. A cryotherapeutic device configured in accordance with a particular example of the present technology can include an elongated shaft having distal portion and a supply lumen along at least a portion of the shaft. The shaft can be configured to locate the distal portion intravascularly at a treatment site proximate a renal artery or renal ostium. The supply lumen can be configured to receive a liquid refrigerant. The cryotherapeutic device can further include a cooling assembly at the distal portion of the shaft. The cooling assembly can include an applicator in fluid communication with the supply lumen and configured to deliver cryotherapeutic cooling to nerves proximate the target site when the cooling assembly is in a deployed state.

WO2011/082278 is directed to a cryotherapy balloon catheter that includes a compliant cryotherapy balloon comprising a distal balloon section dimensioned for placement within a renal artery and a proximal balloon section dimensioned to abut against an ostium of the renal artery and extend into at least a portion of the abdominal aorta. The compliant balloon has a diameter that varies non-uniformly along a length of the compliant balloon, such that a diameter at the proximal balloon section is larger than a diameter of the distal balloon section. The cryotherapy balloon catheter may be configured to deliver cryogenic therapy to at least the ostium of the renal artery sufficient to irreversibly terminate renal sympathetic nerve activity, such as by causing neurotmesis of renal nerve fibers and ganglia at the ostium of the renal artery.

EP2387964 provides a method and system for cryogenically ablating large areas of tissue within the left atrium. In an exemplary embodiment a cryotherapy device includes a catheter body having a substantially fixed diameter, a proximal end and a distal end; a first lumen for permitting passage of a cooling fluid from the proximal end to the distal end; a second lumen permitting return of the cooling fluid from the distal end to the proximal end; and an ablation element expandable from a first diameter that is substantially the same as the diameter of the catheter body to a second diameter that is at least twice the diameter of the catheter body, the ablation element having a surface portion that conforms to the uneven surface topography of the cardiac tissue.

### SUMMARY

The objective of the present invention is to provide a new tumor cryoablation catheter, wherein, a gas intake pipeline extends into the balloon; the space in the balloon ensures that the refrigerant can be fully released to enhance the heat exchange of the refrigerant and make the freezing effect better. The distal end of the new the tumor cryoablation catheter is in a balloon structure. When the balloon is filled, the distal end of the balloon has minimal deformation, thereby ensuring that the distal surface of the balloon is flat, avoiding a point contact between the apparatus and the tissues of human, expanding the freezing range, enhancing the freezing effect and providing a better effect for application to a large-sized tumor.

The object of the present invention is achieved by a device as disclosed in appended independent claim 1. Further preferred embodiments are disclosed in the dependent claims.

The object of the present invention is further achieved by the following technical solutions:
Preferably, the heat insulating layer realizes insulation through a vacuum gap formed between a vacuum tube and the pipelines.
Preferably, an elastic modulus of the distal surface of the balloon is larger than an elastic modulus of the proximal portion.
Preferably, the elastic modulus of the distal surface is more than twice of the elastic modulus of the proximal portion.
Preferably, the distal surface and the proximal portion of the balloon are made of identical materials, and a wall thickness of the distal surface is greater than that of the proximal portion.
Preferably, the distal surface of the balloon is fixedly connected with a reinforcing block; the reinforcing block is made of inelastic material and fixed on an outer surface or an inner surface of the distal end of the balloon.

In the invention, the balloon is provided with a support structure; the distal end of the balloon is fixedly connected with a distal end of the support structure.

Preferably, a length of the support structure is less than a total length of the balloon; the support structure comprises a plurality of inverted "L"-shaped rods, and the inverted "L"-shaped rods are distributed around an axis of the balloon evenly and annularly; a top of each inverted "L"- shaped rod is fixedly connected with the distal end of the balloon.

Preferably, the inverted "L"-shaped rods are made of hard material. In the invention, the support structure is a tubular net; and one portion of the support structure, which corresponds to the distal surface, is a flat surface.

Preferably, the portion of the support structure, which corresponds to the distal surface, is made of inelastic material and is disposed at and combined with the inner wall of the distal portion of the balloon.

Preferably, a density of one portion of the support structure, which corresponds to the distal surface of the balloon, is greater than a density of another portion of the support structure, which corresponds to the proximal portion of the balloon.

In the invention, a diameter of a wire of the net at one portion of the support structure, which corresponds to the distal surface of the balloon, is greater than a diameter of a wire of the net at another portion of the support structure, which corresponds to the proximal portion of the balloon.

Preferably, the gas intake pipeline is disposed coaxially inside the gas return pipeline; or the gas return pipeline is arranged parallel with the gas intake pipeline.

Preferably, the refrigerant is liquid nitrogen.

Compared with the prior art, the beneficial effects of the present invention are mainly as follows:
1. According to the new tumor cryoablation catheter of the present invention, the distal surface of the balloon is a flat surface, and by configuring the elastic modulus of the distal surface to be more than twice of the elastic modulus of the proximal portion, or by configuring the reinforcing block and the support structure, it is ensured that the deformation degree of the distal end of the balloon is far less than the deformation degree of the proximal end of the balloon. When the balloon body is in a state of being filled, the distal end of the balloon generates little deformation and can maintain its preset shape, thereby ensuring that the distal surface of the balloon is flat. During an operation, the distal surface of the balloon presses against the diseased tissues of human body, and the contact area for freezing is large. For a small tumor, the entire range of the diseased tissues can be covered at one time, thereby increasing the range of cryoablation.
2. According to the new tumor cryoablation catheter of the present invention, the distal surface of the balloon is a flat surface, which ensures a surface contact between the distal end of the balloon and the diseased tissues of human body. The apparatus presses against the tissues more closely, thereby increasing the effective freezing area and enhancing the freezing effect.
3. According to the new tumor cryoablation catheter of the present invention, the distal surface of the balloon serves as a cryoablation area, and the distal surface contacts with the diseased tissues, therefore the operation is more convenient, simple and safe. The proximal end of the balloon expands after the high-pressure refrigerant is input, and the expansion of the proximal portion of the balloon caused by the high-pressure refrigerant can form a J-T (Joule-Thomson) effect at the proximal portion of the balloon, thereby enhancing the freezing effect.
4. According to the new tumor cryoablation catheter of the present invention, the elastic modulus of the distal end of the balloon is greater than the elastic modulus of the proximal portion of the balloon, which ensures that the distal deformation is less than the deformation of the proximal portion. Therefore, the distal end has a large hardness, and it is easy for the instrument to pass through some narrow and curved diseased sites, thereby making it easy for the operator to operate.

### BRIEF DESCRIPTION OF THE DRAWINGS

Fig.1 is a schematic view illustrating the distal end of the new tumor cryoablation catheter of the present invention;
Fig.2 is a schematic view illustrating the proximal end of the new tumor cryoablation catheter of the present invention;
Figs. 3A to 3B are schematic views showing different structures of the balloon of the new tumor cryoablation catheter of the present invention;
Fig.4A is a schematic view showing the connection between the balloon and the distal ends of the gas intake pipeline, the gas return pipeline and the vacuum tube of the new tumor cryoablation catheter of the present invention; FIG. 4B is a cross-sectional view of FIG. 4A along the line AA;
Figs.5A and 5B are schematic views showing a combination of the balloon and a support structure of the new tumor cryoablation catheter, wherein, Fig. 5B is a cross-sectional view of FIG. 5A along the line BB;
Figs. 6A to 6C are schematic views illustrating different support structures in the balloon of the new tumor cryoablation catheter of the present invention.

### DETAILED DESCRIPTION OF THE PREFERRED EMBODIMENTS

To make the objectives, technical schemes, and advantages of the present invention clearer, the present disclosure will be further described in more details with reference to the accompanying figures and embodiments.

The proximal end in the present disclosure refers to the end near the surgeon, and the distal end refers to the end far away from the surgeon.

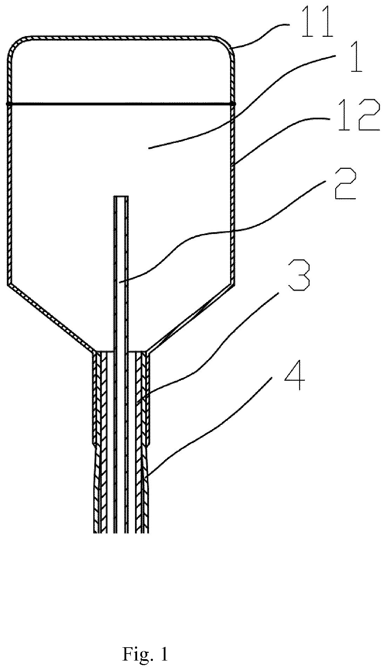

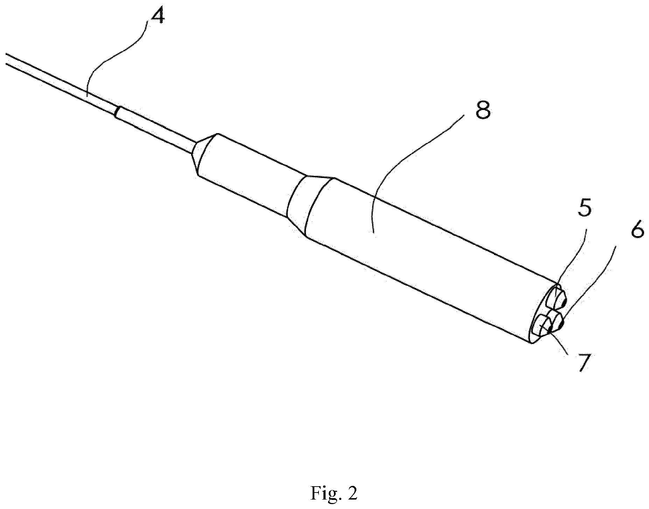

The first preferred embodiment:
As shown in Figs. 1 and 2, a new tumor cryoablation catheter includes a balloon 1 and pipelines connected with the proximal end of the balloon 1. The pipelines are covered with a heat insulating layer. The pipelines include a gas intake pipeline 2 and a gas return pipeline 3. A distal end of the gas intake pipeline 2 extends into the balloon 1. The heat insulating layer realizes insulation through a vacuum gap formed between the vacuum tube 4 and the pipelines. The new tumor cryoablation catheter of the present invention further includes a handle 8, a gas inlet joint 5 hermetically connected with the proximal end of the gas inlet pipeline 2, a gas return joint 6 hermetically connected with the proximal end of the gas return pipeline 3, and a vacuum joint 7 hermetically connected with the distal end of the vacuum tube 4. The proximal end of the balloon 1 is hermetically connected with the distal end of the gas return pipeline 3 and the distal end of the vacuum tube 4. The vacuum tube 4 is disposed outside the gas intake pipeline 2 and the gas return pipeline 3. The gas intake pipeline 2 is coaxially disposed inside the gas return pipeline 3; the distal end of the vacuum tube 4 is hermetically connected with the distal end of the gas return pipeline 3; the proximal end of the vacuum tube 4 is hermetically connected with the proximal end of the gas return pipeline 3; the handle 8 is coated outside the vacuum tube 4; the gas inlet joint 5, the gas return joint 6 and the vacuum joint 7 are arranged at the proximal end of the handle 8 in a straight line, in a triangle or in any other shape; and the gas intake pipeline 2 is arranged inside the gas return pipeline 3.

In one of the embodiments, the balloon 1 comprises a distal surface 11 and a proximal portion 12, which are fixedly connected together. The distal surface 11 and the proximal portion 12 have the same wall thickness, but are made of different materials. The elastic modulus of the distal surface 11 is larger than the elastic modulus of the proximal portion 12, therefore, the deformation degree of the distal surface 11 is smaller than the deformation degree of the proximal portion 12. The distal surface 11 is not easy to deform under an external force, thus ensures that, in the process of ablation and when the gas intake pipeline 2 inputs refrigerant to expand the balloon 1, the distal surface 11 is expanded to form a plane. The heat insulating layer is disposed outside the gas return pipeline 3 and the gas intake pipeline 2, and the heat insulating layer effectively realizes insulation protection through the vacuum gap formed between the vacuum tube 4 and the return pipeline 3, thereby avoiding ultra-low temperature conduction of the surface of the gas return pipeline 3.

In the process of ablation, when the refrigerant enters the balloon 1 from the gas intake pipeline 2, the input refrigerant expands the balloon 1, and the distal surface 11 is spread to form a plane at the distal end of the balloon 1, and the proximal portion 12 of the balloon 1 bulges to form a not-depicted hemispherical shape, then the refrigerant flows back through the gas return pipeline 3. Since the elastic modulus of the distal surface 11 is configured to be more than twice of the elastic modulus of the proximal portion, the shape of the distal surface 11will be less affected by gas pressure, and the preset shape can be well maintained, that is, the distal surface 11 is a plane. Serving as a contact surface, the distal surface 11 presses against the diseased site of the human body, so that the contact surface is large enough to completely cover the tumor surface with a general size at one time, thereby reducing the number of freezing operations, further reducing the damages to the human body in the freezing process, reducing complications and improving the survivability of the patients. At the same time, as the elastic modulus of the distal surface 11 in the state of being filled is large, the distal surface 11 is not easy to deform under external forces, which makes the distal surface 11 of the balloon 1 maintain flat. A good fit can be achieved when the distal surface 11 contacts with the human tissue, and the contact is a surface contact, not a point contact. The surface contact can effectively enhance the freezing range and freezing effects, and ensure the effectiveness of cryoablation. Since the material of the distal surface 11 has a large elastic modulus, the deformability of the material is small, and during operation, it is easy for the cryoablation catheter to pass some narrow diseased sites, thereby making it easy for the operator to operate.

What's more, when the balloon 1 is expanded by the high-pressure refrigerant, the proximal end of the balloon 1 expands after the refrigerant is input, and the expansion of the proximal portion 12 of the balloon 1 caused by the high-pressure refrigerant can form a J-T (Joule-Thomson) effect at the proximal portion 12 of the balloon 1, thereby enhancing the freezing effect.

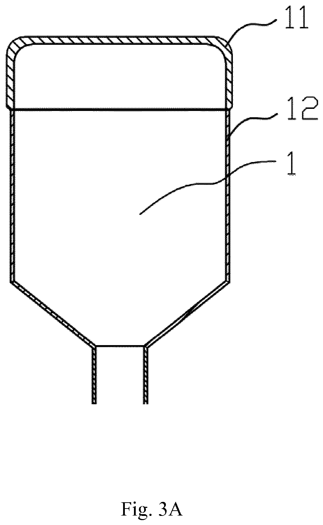

In some of embodiments, as shown in Fig. 3A, the distal surface 11 of the balloon 1 and the proximal portion 12 of the balloon 1 are made of identical materials, and a wall thickness of the distal surface 11 is greater than that of the proximal portion 12. The distal surface 11 is fixedly connected with the proximal portion 12. The thickness of the wall of the distal surface 11 is large, so the wall has larger hardness, and under external forces, the distal surface can resist deformation and maintain a preset shape, namely, the distal surface 11 of the balloon 1 maintains flat. The distal surface 11 and the proximal portion 12 are made of identical material, and the process of the fixed connection is relatively simple and reliable, and is easy to implement.

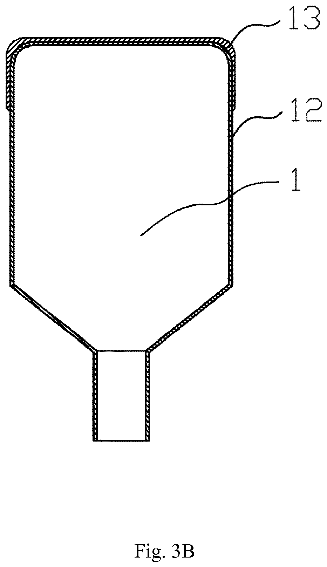

As shown in Fig. 3B, the distal surface of the balloon 1 is fixedly connected with a reinforcing block 13, which is made of inelastic material and fixed on an outer surface or an inner surface of the distal end of the balloon 1. The reinforcing block 13 is configured to enable the distal surface 11 of the balloon 1 to be less deformed than the proximal portion 12. When the inside of the balloon 1 is subjected to gas pressure, the reinforcing block 13 can effectively stop the distal surface 11 of the balloon 1 from deforming, thus the distal surface 11 of the balloon 1 can maintain its original shape. What's more, the manufacturing process of the structure is relatively simple and easy to implement, thereby reducing production costs.

The second preferred embodiment:
As shown in Fig. 2, Figs. 4A to 6C, a new tumor cryoablation catheter comprises a balloon 1, a gas intake pipeline 2, a gas return pipeline 3, a vacuum tube 4, a handle 8, a gas inlet joint 5 hermetically connected with a proximal end of the gas intake pipeline 2, a gas return joint 6 hermetically connected with the proximal end of the gas return pipeline 3, a vacuum joint 7 hermetically connected with the proximal end of the vacuum tube 4. A proximal end of the balloon 1 is hermetically connected with the distal end of the gas return pipeline 3 and the distal end of the vacuum tube 4. The vacuum tube 4 is disposed outside the gas intake pipeline 2 and outside the gas return pipeline 3. The gas intake pipeline 2 is disposed outside the gas return pipeline 3 and arranged parallel with the gas return pipeline 3. The distal end of the vacuum tube 4 is hermetically connected with the distal end of the gas intake pipeline 2 and the distal end of the gas return pipeline 3; the proximal end of the vacuum tube 4 is hermetically connected with the proximal end of the gas intake pipeline 2 and the proximal end of the gas return pipeline 3; the handle 8 is coated outside the vacuum tube 4; the gas inlet joint 5, the gas return joint 6 and the vacuum joint 7 are arranged at the proximal end of the handle 8. The balloon 1 is provided with a support structure 9, whose length is less than or equal to the total length of the balloon 1. The distal end of the balloon 1 is fixedly connected with the distal end of the support structure 9. The distal surface 11 of the body 1 is a flat surface, and the balloon 1 is flexible and foldable.

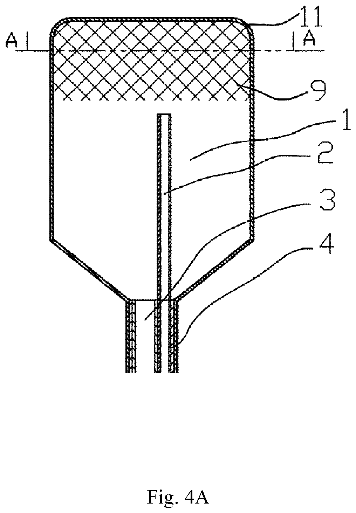

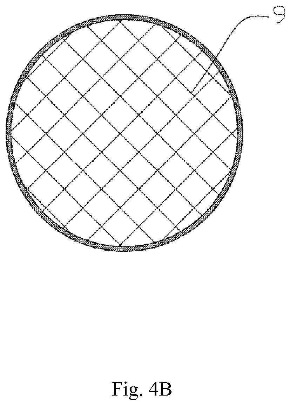

As shown in Fig. 4A to 4B, the length of the support structure 9 is less than the total length of the balloon 1; the support structure 9 is a tubular net; and a portion of the support structure 9, which corresponds to the distal surface 11 of the balloon 1, is a flat surface. The distal end of the support structure 9 is fixedly connected with the distal end of the balloon 1, and the portion of the support structure 9, which corresponds to the distal surface 11, is made of inelastic material and is disposed at and combined with the inner wall of the distal portion of the balloon 1. The support structure 9 is arranged at the distal portion of the balloon 1, thereby reducing the deformation degree of the distal surface 11 of the balloon 1, and making the deformation degree of the distal surface 11 of the balloon 1 less than that of the proximal portion of the balloon. When the balloon 1 is subjected to the pressure of refrigerant, the deformation of the distal surface is quite small, and the distal surface maintains its original shape, and the distal surface 11 of the balloon 1 maintains flat.

In an actual operation, when the balloon 1 is filled, since the support structure 9 arranged at the distal portion of the balloon 1 has a larger hardness, the shape of the distal portion of the balloon 1 can be maintained well, so as to maintain a surface contact between the distal surface 11 and the human body, thereby enhancing the treatment effect of cryoablation and increasing the freezing range. Since the support structure 9 is a net, it is easy to be folded and is convenient for doctors to operate.

In this embodiment, the gas intake pipeline 2 is arranged outside the gas return pipeline 3. In the actual freezing process, the refrigerant in the gas intake pipeline is released into the balloon 1 and exchanges heat with diseased site of the human body, then flows into the gas return pipelines, so the temperature in the gas return pipeline is higher than that in the gas intake pipeline. The structure shown in Fig. 4A can reduce the influence of the temperature in the gas return pipeline on the temperature in the gas intake pipeline, and can reduce the loss of refrigeration capacity. The vacuum tube 4 is arranged outside the gas intake pipeline 2 and the gas return pipeline 3, thus reduces heat loss and ensures that the temperature of the outer surface of the vacuum tube 4 is at a normal temperature, thereby avoiding damages to non- pathological area of the human body.

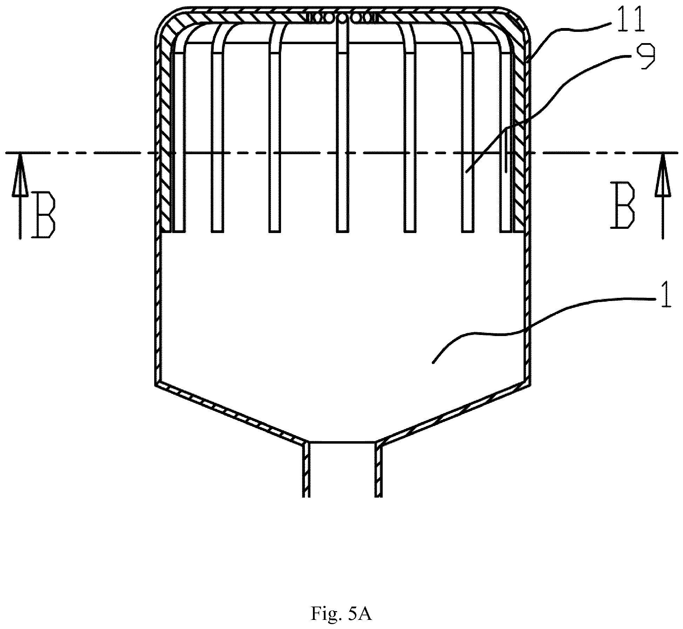

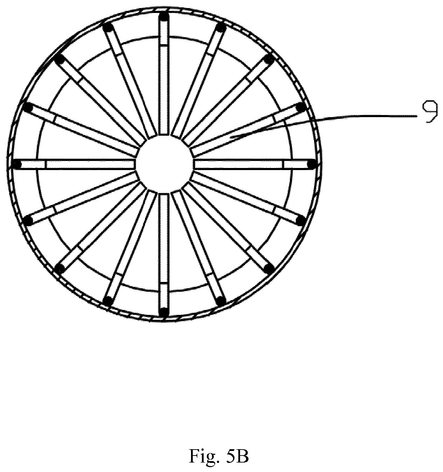

In some embodiments, as shown in Figs. 5A and 5B, the support structure 9 comprises a plurality of inverted "L"-shaped rods, and the inverted "L"-shaped rods are distributed around the axis of the balloon 1 evenly and annularly, and are arranged to be a columnar grid structure. The inverted "L"-shaped rods are made of hard material, and the top of each inverted "L"- shaped rod is fixedly connected with the distal end of the balloon 1. The length of the support structure 9 is less than the total length of the balloon 1, and the support structure 9 increases the hardness of the distal portion of the balloon 1 and reduces the deformation degree of the distal portion of the balloon 1, therefore the ability to resist deformation of the distal portion of the balloon 1 is enhanced, and the distal surface 11 of the balloon 1 can maintain the flat surface well. The manufacturing process of the support structure 9 is simple and easy to implement, and the support structure 9 is convenient to operate during operation.

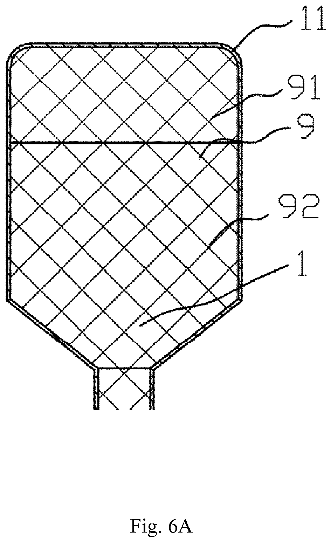

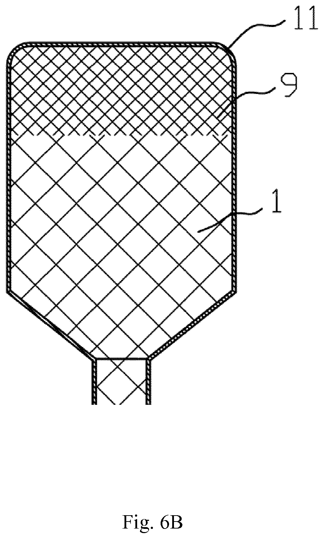

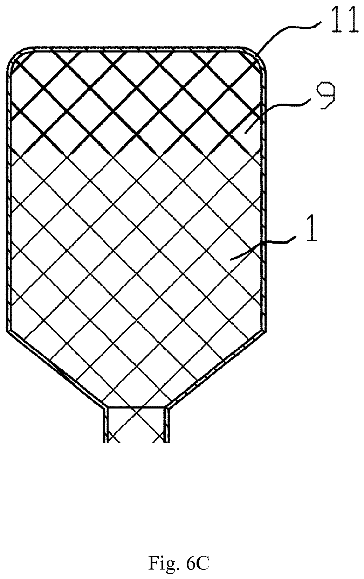

In some embodiments, as shown in Figs. 6A to 6C, the length of the support structure 9 is equal to the total length of the balloon 1; the support structure 9 is in a net-shaped structure; the deformation degree of the distal portion of the net structure is less than the deformation degree of the proximal portion thereof; and the distal end of the support structure 9 is fixedly connected with the distal end of the balloon 1, which ensures the ability of the distal surface 11 of the balloon 1 to maintain its original shape, so that the distal surface 11 of the balloon 1 is a flat surface to press against the diseased tissues of the human. As shown in Fig. 6A, the support structure 9 includes a low elastic portion 91 corresponding to the distal surface 11 of the balloon 1 and a high elastic portion 92 corresponding to the proximal portion 12 of the balloon 1. The low elastic portion 91 is fixedly connected with the high elastic portion 92, and the low elastic portion 91 and the high elastic portion 92 are made of different material. The material elongation of the low elastic portion 91 is less than that of the high elastic portion 92, and the ability of the low-elastic portion 91 to resist deformation is stronger than that of the high-elastic portion 92. This configuration can ensure that, when the balloon 1 is subjected to internal pressure, the balloon maintains the original preset shape, and the distal surface 11 under pressure will not present an arc surface. As shown in Fig. 6B, the support structure 9 is in a net-shaped structure, and its distal end is fixedly connected with the distal end of the balloon 1. The density of one portion of the support structure 9, which corresponds to the distal surface 11 of the balloon 1, is greater than the density of another portion of the support structure 9, which corresponds to the proximal portion 12 of the balloon 1. The distal portion of the support structure 9 of this structure has a good hardness and has strong ability to resist deformation, thus ensures the distal surface 11 of the balloon 1 to maintain flat. The structure is convenient for the operator to operate and can enhance the effect of cryoablation. As shown in FIG. 6C, the support structure 9 is in a net-shaped structure, and its distal end is fixedly connected with the distal end of the balloon 1. The diameter of the wire of the net at one portion of the support structure 9, which corresponds to the distal surface 11 of the balloon 1, is greater than the diameter of the wire of the net at another portion of the support structure 9, which corresponds to the proximal portion 12 of the balloon 1. The larger the diameter of the wire of the net is, the smaller the deformation degree of the net structure is, which is more beneficial to resist deformation. The distal portion of the support structure 9 of this structure has a large hardness and ensures that the distal surface 11 of the balloon 1 can maintain flat.

The structures of the gas inlet joint 5, the gas return joint 6, the vacuum joint 7 and the handle 8 are the same as those of the first preferred embodiment, and will not be described herein.

It should be noted that the above mentioned are only preferred embodiments of the present invention, but not intended to limit the implementations of the present invention. All modifications, equivalents, improvements and so on made within the principle of the present disclosure should be contained within the scope of the appended claims.

## Claims

1. Anew tumor cryoablation catheter, comprising a balloon (1) and pipelines connected with a proximal end of the balloon (1); the pipelines are covered with a heat insulating layer; the pipelines include a gas intake pipeline (2) and a gas return pipeline (3); a distal end of the gas intake pipeline (2) extends into the balloon (1); wherein
the balloon (1) comprises a distal surface (11) disposed at a distal end of the balloon; the balloon (1) is provided with a support structure (9); the distal end of the balloon (1) is fixedly connected with a distal end of the support structure (9); the support structure (9) is a tubular net; and one portion of the support structure (9), which corresponds to the distal surface (11), is a flat surface; in a process of ablation, refrigerant input from the gas intake pipeline (2) expands the balloon (1), and the distal surface (11) is spread to form a flat surface at the distal end of the balloon (1) and the refrigerant flows back through the gas return pipeline (3);
and **characterized in that** a diameter of a wire of the net at one portion of the support structure (9), which corresponds to the distal surface (11) of the balloon (1), is greater than a diameter of a wire of the net at another portion of the support structure (9), which corresponds to the proximal portion (12) of the balloon (1); a proximal portion (12) of the balloon (1) bulges to form a hemispherical shape.

2. The new tumor cryoablation catheter according to claim 1, **characterized in that**, the heat insulating layer realizes insulation through a vacuum gap formed between a vacuum tube (4) and the pipelines.

3. The new tumor cryoablation catheter according to claim 1, **characterized in that**, an elastic modulus of the distal surface (11) of the balloon (1) is larger than an elastic modulus of the proximal portion (12).

4. The new tumor cryoablation catheter according to claim 3, **characterized in that**, the elastic modulus of the distal surface (11) is more than twice of the elastic modulus of the proximal portion (12).

5. The new tumor cryoablation catheter according to claim 1, **characterized in that**, the distal surface (11) and the proximal portion (12) of the balloon (1) are made of identical materials, and a wall thickness of the distal surface (11) is greater than that of the proximal portion (12).

6. The new tumor cryoablation catheter according to claim 1, **characterized in that**, the portion of the support structure (9), which corresponds to the distal surface (11), is made of inelastic material and is disposed at and combined with the inner wall of the distal portion (11) of the balloon (1).

7. The new tumor cryoablation catheter according to claim 1, **characterized in that**, a density of one portion of the support structure (9), which corresponds to the distal surface (11) of the balloon (1), is greater than a density of another portion of the support structure (9), which corresponds to the proximal portion (12) of the balloon (1).

8. The new tumor cryoablation catheter according to claim 1, **characterized in that**, the gas intake pipeline (2) is disposed coaxially inside the gas return pipeline (3); or the gas return pipeline (3) is arranged parallel with the gas intake pipeline (2).

## Patentansprüche

1. Neuer Tumor-Kryoablationskatheter, umfassend einen Ballon (1) und mit einem proximalen Ende des Ballons (1) verbundene Rohrleitungen, wobei die Rohrleitungen mit einer Wärmeisolierungsschicht bedeckt sind, wobei die Rohrleitungen eine Gaszuleitung (2) und eine Gasrückleitung (3) aufweisen, wobei sich ein distales Ende der Gaszuleitung (2) in den Ballon (1) erstreckt, wobei der Ballon (1) eine distale Fläche (11) umfasst, die an einem distalen Ende des Ballons angeordnet ist, wobei der Ballon (1) mit einer Stützstruktur (9) versehen ist, wobei das distale Ende des Ballons (1) fest mit einem distalen Ende der Stützstruktur (9) verbunden ist, wobei die Stützstruktur (9) ein rohrförmiges Netz ist und ein Abschnitt der Stützstruktur (9), der der distalen Fläche (11) entspricht, eine flache Fläche ist, wobei die Einleitung von Kühlmittel aus der Gaszuleitung (2) bei einem Ablationsvorgang den Ballon (1) expandiert und die distale Fläche (11) ausgebreitet wird, um eine flache Fläche an dem distalen Ende des Ballons (1) zu bilden, und das Kühlmittel durch die Gasrückleitung (3) zurückströmt, **dadurch gekennzeichnet, dass** ein Durchmesser eines Drahts des Netzes an einem Abschnitt der Stützstruktur (9), der der distalen Fläche (11) des Ballons (1) entspricht, größer als ein Durchmesser eines Drahts des Netzes an einem anderen Abschnitt der Stützstruktur (9) ist, der dem proximalen Abschnitt (12) des Ballons (1) entspricht,
ein proximaler Abschnitt (12) des Ballons (1) sich wölbt, um eine Halbkugel zu bilden.

2. Neuer Tumor-Kryoablationskatheter nach Anspruch 1, **dadurch gekennzeichnet, dass** die Wärmeisolierungsschicht für Isolierung über eine zwischen einem Vakuumschlauch (4) und den Rohrleitungen ausgebildete Vakuumstrecke sorgt.

3. Neuer Tumor-Kryoablationskatheter nach Anspruch 1, **dadurch gekennzeichnet, dass** ein Elastizitätsmodul der distalen Fläche (11) des Ballons (1) größer als ein Elastizitätsmodul des proximalen Abschnitts (12) ist.

4. Neuer Tumor-Kryoablationskatheter nach Anspruch 3, **dadurch gekennzeichnet, dass** der Elastizitätsmodul der distalen Fläche (11) mehr als das Doppelte des Elastizitätsmoduls des proximalen Abschnitts (12) beträgt.

5. Neuer Tumor-Kryoablationskatheter nach Anspruch 1, **dadurch gekennzeichnet, dass** die distale Fläche (11) und der proximale Abschnitt (12) des Ballons (1) aus identischen Materialien hergestellt sind und eine Wanddicke der distalen Fläche (11) größer als die des proximalen Abschnitts (12) ist.

6. Neuer Tumor-Kryoablationskatheter nach Anspruch 1, **dadurch gekennzeichnet, dass** der Abschnitt der Stützstruktur (9), der der distalen Fläche (11) entspricht, aus unelastischem Material hergestellt und an der Innenwand des distalen Abschnitts (11) des Ballons (1) angeordnet und mit dieser kombiniert ist.

7. Neuer Tumor-Kryoablationskatheter nach Anspruch 1, **dadurch gekennzeichnet, dass** eine Dichte eines Abschnitts der Stützstruktur (9), der der distalen Fläche (11) des Ballons (1) entspricht, größer als eine Dichte eines anderen Abschnitts der Stützstruktur (9) ist, der dem proximalen Abschnitt (12) des Ballons (1) entspricht.

8. Neuer Tumor-Kryoablationskatheter nach Anspruch 1, **dadurch gekennzeichnet, dass** die Gaszuleitung (2) koaxial in der Gasrückleitung (3) angeordnet ist oder die Gasrückleitung (3) parallel zu der Gaszuleitung (2) angeordnet ist.

## Revendications

1. Nouveau cathéter de cryoablation de tumeur, comprenant un ballonnet (1) et des canaux raccordés à une extrémité proximale du ballonnet (1), les canaux étant recouverts d'une couche d'isolation thermique ; les canaux comprenant un canal d'admission de gaz (2) et un canal de retour de gaz (3) ; une extrémité distale du canal d'admission de gaz (2) s'étendant dans le ballonnet (1) ; le ballonnet (1) comprenant une surface distale (11) disposée à une extrémité distale du ballonnet ; le ballonnet (1) étant muni d'une structure de support (9), l'extrémité distale du ballonnet (1) étant raccordée de manière fixe à une extrémité distale de la structure de support (9), la structure de support (9) étant un filet tubulaire ; et une partie de la structure de support (9), qui correspond à la surface distale (11), étant une surface plane ; dans un processus d'ablation, l'entrée de réfrigérant provenant du canal d'admission de gaz (2) dilatant le ballonnet (1), et la surface distale (11) étant étalée pour former une surface plane à l'extrémité distale du ballonnet (1), et le réfrigérant revenant par le canal de retour de gaz (3) ; et **caractérisé en ce qu'**un diamètre d'un fil du filet au niveau d'une partie de la structure de support (9), qui correspond à la surface distale (11) du ballonnet (1), est supérieur à un diamètre d'un fil du filet au niveau d'une autre partie de la structure de support (9), qui correspond à la partie proximale (12) du ballonnet (1) ;
une partie proximale (12) du ballonnet (1) est bombée pour former une forme hémisphérique.

2. Nouveau cathéter de cryoablation de tumeur selon la revendication 1, **caractérisé en ce que**, la couche d'isolation thermique réalise une isolation à travers un espace sous vide formé entre un tube à vide (4) et les canaux.

3. Nouveau cathéter de cryoablation de tumeur selon la revendication 1, **caractérisé en ce qu'**un module d'élasticité de la surface distale (11) du ballonnet (1) est supérieur à un module d'élasticité de la partie proximale (12).

4. Nouveau cathéter de cryoablation de tumeur selon la revendication 3, **caractérisé en ce que**, le module d'élasticité de la surface distale (11) est plus de deux fois supérieur au module d'élasticité de la partie proximale (12).

5. Nouveau cathéter de cryoablation de tumeur selon la revendication 1, **caractérisé en ce que**, la surface distale (11) et la partie proximale (12) du ballonnet (1) sont constituées de matériaux identiques, et une épaisseur de paroi de la surface distale (11) est supérieure à celle de la partie proximale (12).

6. Nouveau cathéter de cryoablation de tumeur selon la revendication 1, **caractérisé en ce que** la partie de la structure de support (9), qui correspond à la surface distale (11), est constituée d'un matériau inélastique et est disposée au niveau de la paroi interne de la partie distale (11) du ballonnet (1) et combinée avec celle-ci.

7. Nouveau cathéter de cryoablation de tumeur selon la revendication 1, **caractérisé en ce que**, une densité d'une partie de la structure de support (9), qui correspond à la surface distale (11) du ballonnet (1), est supérieure à une densité d'une autre partie de la structure de support (9), qui correspond à la partie proximale (12) du ballonnet (1).

8. Nouveau cathéter de cryoablation de tumeur selon la revendication 1, **caractérisé en ce que**, le canal d'admission de gaz (2) est disposé coaxialement à l'intérieur du canal de retour de gaz (3) ; ou le canal de retour de gaz (3) est agencé parallèlement au canal d'admission de gaz (2).
